(19) Europäisches Patentamt
European Patent Office
Office européen des brevets

(11) Veröffentlichungsnummer: **0 281 826**
**A2**

(12) # EUROPÄISCHE PATENTANMELDUNG

(21) Anmeldenummer: **88102508.4**

(51) Int. Cl.⁴: **C07C 99/00** , C07C 101/30

(22) Anmeldetag: **20.02.88**

(30) Priorität: **11.03.87 DE 3707719**

(43) Veröffentlichungstag der Anmeldung:
**14.09.88 Patentblatt 88/37**

(84) Benannte Vertragsstaaten:
**AT BE CH DE ES FR GB GR IT LI LU NL SE**

(71) Anmelder: **Degussa Aktiengesellschaft**
**Weissfrauenstrasse 9**
**D-6000 Frankfurt am Main 1(DE)**

(72) Erfinder: **Häusler, Johannes, Dr.**
**Geblergasse 13-5**
**A-1170 Wien(AT)**

(54) **Verfahren zur Herstellung von (R)-4-Amino-3-hydroxybutansäure (GABOB).**

(57) (R)-4-Amino-3-hydroxybutansäure wird aus dem Hydrochlorid des Methyl-oder Ethylesters von (2S, 4R)-4-Hydroxyprolin über die Zwischenstufen des entsprechenden, nicht isolierten (R)-4-Hydroxy-1-pyrrolin-2-carbonsäureesters und des isolierten (R)-4-Hydroxy-2-pyrrolidons hergestellt. Nach diesem Verfahren ist die pharmakologisch interessante (R)-4-Amino-3-hydroxybutansäure in hoher Ausbeute erhältlich.

EP 0 281 826 A2

**Verfahren zur Herstellung von (R)-4-Amino-3-hydroxybutansäure (GABOB)**

Gegenstand der Erfindung ist ein Verfahren zur Herstellung von (R)-4-Amino-3-hydroxybutansäure, welches dadurch gekennzeichnet ist, daß man

a) ein (2S, 4R)-4-Hydroxyprolinester-hydrochlorid der Formel

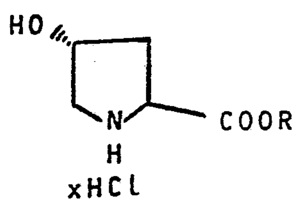

(I),

in der R einen Methyl-oder Ethylrest bedeutet, durch Behandeln in wasserfreiem Medium mit zuerst einem Alkalimetallmethoxid oder -ethoxid, dann t-Butylhypochlorit und schließlich Triethylamin in den (R)-4-Hydroxy-1-pyrrolin-2-carbonsäureester der Formel

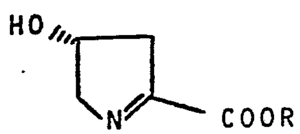

(II),

in der R wieder die bereits angegebene Bedeutung hat, umwandelt,

b) diesen ohne weitere Reinigung in einer Alkalimetallhydroxid-Lösung auflöst, die Lösung mit Wasserstoffperoxid versetzt, das Reaktionsgemisch durch langsame Zugabe einer Mineralsäure ansäuert und anschließend entsalzt und durch Einengen das gebildete (R)-4-Hydroxy-2-pyrrolidon der Formel

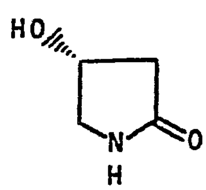

(III)

gewinnt und

c) dieses mit einer Mineralsäure zu (R)-4-Amino-3-hydroxybutansäure der Formel

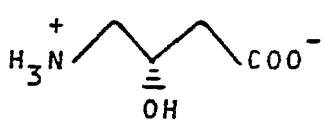

(IV)

hydrolysiert.

Da das als Ausgangsmaterial dienende 4-Hydroxyprolin-Derivat der Formel (I) seinerseits auf relativ einfache Weise aus dem natürlich vorkommenden L-4-Hydroxyprolin hergestellt werden kann, eröffnet das erfindungsgemäße Verfahren letztlich einen relativ einfachen und kostengünstigen Weg von dem letzteren zu (R)-4-Amino-3-hydroxybutansäure (GABOB).

Die nach dem erfindungsgemäßen Verfahren in hoher Ausbeute erhältliche (R)-4-Amino-3-hydroxybutansäure selbst, wie auch das aus ihr leicht zugängliche Trimethylbetain L-Carnitin sind pharmakologisch recht interessante Substanzen.

Bei der praktischen Durchführung des erfindungsgemäßen Verfahrens muß zunächst aus dem (2S, 4R)-4-Hydroxyprolinester-hydrochlorid der Formel (I) durch Behandeln mit einem Alkalimetallmethoxid oder -ethoxid der (2S, 4R)-4-Hydroxyprolinester freigesetzt werden. Besonders geeignet sind hierzu die Methoxide und Ethoxide des Natriums und Kaliums, die zweckmäßigerweise als Lösung in dem entsprechenden Alkohol eingesetzt werden. Bevorzugt wird die Verwendung einer Lösung von Natriummethoxid in absolutem Methanol. Das Alkalimetallmethoxid oder -ethoxid wird vorzugsweise in der 1-bis 1,05-fachen, insbesondere in der 1-bis 1,01-fachen, molaren Menge des eingesetzten (2S, 4R)-4-Hydroxyprolinester hydrochlorids angewandt. Die Temperatur sollte zweckmäßigerweise zwischen 10 und 30°C, vorzugsweise zwischen 15 und 25°C, liegen.

Da die nachfolgende Behandlung des freigesetzten (2S, 4R)-4-Hydroxyprolinesters mit t-Butylhypochlorit zweckmäßigerweise in einem eher unpolaren Lösungsmittel vorgenommen werden sollte, ist es vorteilhaft, wenn nun unter schonenden Bedingungen der Alkohol möglichst vollständig entfernt und der Rückstand in absolutem Tetrahydrofuran oder 1,2-Dimethoxyethan oder in einer Mischung aus einem dieser Ether und absolutem Diethylether aufgenommen wird. Das t-Butylhypochlorit wird vorzugsweise in der 1-bis 1,05-fachen, insbesondere in der 1-bis 1,02-fachen, molaren Menge des ursprünglich eingesetzten (2S, 4R)-4-Hydroxyprolinester-hydrochlorids angewandt. Die Temperatur sollte zweckmäßigerweise zwischen -40 und -20°C gehalten werden.

Wenn die Umsetzung mit dem t-Butylhypochlorit, die der N-Chlorierung des (2S, 4R)-4-Hydroxyprolinesters dient, beendet ist, wird das N-Chlor-Derivat anschließend ohne Zwischenisolierung im gleichen Lösungsmittel(gemisch) mit Triethylamin behandelt. Dabei wird unter Dehydrochlorierung die Doppelbindung zwischen dem Stickstoffatom und dem Kohlenstoffatom in 2-Stellung ausgebildet und es entsteht der (R)-4-Hydroxy-1-pyrrolin-2-carbonsäureester der Formel (II). Das Triethylamin wird dabei zweckmäßigerweise in der 1-bis 1,05-fachen, vorzugsweise in der 1-bis 1,03-fachen, molaren Menge des ursprünglich eingesetzten (2S, 4R)-4-Hydroxyprolinesterhydrochlorids angewandt. Die Temperatur sollte zweckmäßigerweise zwischen -20 und +10°C gehalten werden.

Es ist vorteilhaft, wenn man nach der Zugabe des Triethylamins das Reaktionsgemisch längere Zeit, z.B. über Nacht, bei einer Temperatur zwischen 0 und +10°C stehen läßt. Dann werden die ausgeschiedenen Salze abgetrennt und mit frischem Lösungsmittel(gemisch) nachgewaschen. Die vereinigten Filtrate werden unter vermindertem Druck schonend eingeengt.

Der als Öl zurückbleibende (R)-4-Hydroxy-1-pyrrolin-2-carbonsäureester wird anschließend ohne weitere Reinigung in einer wäßrigen Alkalimetallhydroxid-Lösung, vorzugsweise Natronlauge, aufgenommen und mit einer wäßrigen Lösung von Wasserstoffperoxid versetzt. Dann wird langsam eine Mineralsäure, vorzugsweise Schwefelsäure oder Salzsäure, zugegeben. Das Reaktionsgemisch wird noch einige Zeit bei mäßig hoher Temperatur stehen gelassen, bevor das überschüssige Wasserstoffperoxid, beispielsweise durch Zugabe einer wäßrigen Natriumsulfit-Lösung, zerstört wird. In dieser Reaktionsstufe b) wird das Alkalimetallhydroxid und das Wasserstoffperoxid zweckmäßigerweise in der 1-bis 1,05-fachen, vorzugsweise in der 1-bis 1,03-fachen, molaren Menge des ursprünglich eingesetzten (2S, 4R)-4-Hydroxyprolinesterhydrochlorids und die Mineralsäure in einer dem eingesetzten Alkalimetallhydroxid mindestens äquivalenten Menge angewandt. Die Temperatur sollte in dieser Reaktionsstufe b) zweckmäßgierweise zwischen 20 und 50°C, vorzugsweise zwischen 20 und 40°C, liegen.

Das angesäuerte Reaktionsgemisch wird nun, gegebenenfalls nach einer gewissen Einengung unter vermindertem Druck, durch Behandeln mit einem stark sauren Ionenaustauscher in der $H^+$-Form und mit einem stark basischen Ionenaustauscher in der $HO^-$-Form entsalzt, und die entsalzte Lösung wird unter vermindertem Druck bis zur Trockne eingedampft. Der Rückstand wird vorteilhafterweise mehrmals in absolutem Ethanol aufgenommen und wieder eingeengt, um restliches Wasser zu entfernen, und schließlich mit absolutem Diethylether angerieben. Die erhaltenen Kristalle von (R)-4-Hydroxy-2-pyrrolidon der Formel (III) können zur weiteren Reinigung im Hochvakuum sublimiert werden.

Schließlich wird das (R)-4-Hydroxy-2-pyrrolidon der Formel (III) in überschüssiger Mineralsäure, vorzugsweise Schwefelsäure und insbesondere Salzsäure, hydrolysiert. Die Mineralsäure wird zweckmäßigerweise in der 5-bis 10-fachen, vorzugsweise der 5-bis 6-fachen, molaren Menge des eingesetzten (R)-4-Hydroxy-2-pyrrolidons und in einer Konzentration zwischen 4N und 6N eingesetzt. Die Hydrolysetemperatur kann beispielsweise zwischen 90°C und der Rückflußtemperatur des Reaktionsgemisches liegen. Vorzugsweise erfolgt die Hydrolyse durch Erhitzen unter Rückfluß.

Nach beendeter Hydrolyse kann das Reaktionsgemisch beispielsweise so aufgearbeitet werden, daß man es mit einem stark sauren Ionenaustauscher in der $H^+$-Form behandelt und die (R)-4-Amino-3-hydroxybutansäure mit verdünnter wäßriger Ammoniak-Lösung eluiert. Das Eluat wird dann unter vermindertem Druck zur Trockne eingedampft. Die zurückbleibende (R)-4-Amino-3-hydroxybutansäure der Formel (IV) kann zur weiteren Reinigung, gegebenenfalls unter Zusatz von Aktivkohle, aus einem Wasser/Ethanol-Gemisch umkristallisiert werden.

Die Erfindung wird durch das nachfolgende Beispiel näher erläuert. Die Schmelzpunkte wurden auf einem Heiztischmikroskop nach Kofler bestimmt und die spezifischen Drehungen wurden mit einem

Polarimeter 241 der Firma Perkin-Elmer gemessen.

Beispiel:

Reaktionsstufe a):

7,24 g (40 mMol) scharf getrocknetes (2S, 4R)-4-Hydroxyprolinmethylester-hydrochlorid wurden in einem Kolben unter Umschwenken mit 40,0 ml einer 1N Lösung von Natriummethoxid in wasserfreiem Methanol versetzt. Die entstandene Suspension wurde in einem Rotationsverdampfer bei einer Badtemperatur bis 25°C bis zur öligen Konsistenz des Rückstandes eingeengt. Dieser Rückstand wurde in 120 ml einer Mischung aus absolutem Tetrahydrofuran und absolutem Diethylether (Volumenverhältnis 2:1) aufgenommen und bei einer Temperatur von -40°C unter Rühren mit 4,40 g (40,5 mMol) frisch destilliertem t-Butylhypochlorit versetzt. Die Lösung wurde innerhalb von 45 Minuten auf -20°C kommen gelassen und dann wurden 5,7 ml (41 mMol) wasserfreies Triethylamin zugegeben. Das Reaktionsgemisch wurde noch 1 Stunde lang auf -20°C belassen und dann über Nacht bei ca. +5°C im Kühlschrank aufbewahrt. Das auskristallisierte Triethylamin-hydrochlorid und Natriumchlorid wurden abgesaugt und mit frischem Lösungsmittelgemisch gewaschen. Die vereinigten Filtrate wurden unter vermindertem Druck schonend eingeengt. Von dem in öliger Form zurückbleibenden (R)-4-Hydroxy-1-pyrrolin-2-carbonsäuremethylester wurde eine Probe dünnschichtchromatographisch auf einer DC-Platte mit Kieselgel 60 der Firma Merck unter Verwendung einer Mischung aus Chloroform und Methanol (Volumenverhältnis 9:1) als Laufmittel untersucht. Der $R_f$-Wert betrug 0,36.

Reaktionsstufe b):

Das in der Reaktionsstufe a) zurückgebliebene Öl wurde ohne weitere Reinigung rasch mit 41 ml 1N Natronlauge und 4,1 ml (40 mMol) einer 30 gewichtsprozentigen wäßrigen Wasserstoffperoxid-Lösung versetzt. Innerhalb von 20 Minuten wurden 4,5 ml 10 N Schwefelsäure zugetropft und das Reaktionsgemisch wurde noch 45 Minuten lang auf einer Temperatur von 40°C gehalten. Dann wurde durch Zugabe von wenig Natriumsulfit das überschüssige Wasserstoffperoxid zerstört, die Lösung wurde unter vermindertem Druck etwas eingeengt und dann durch Behandeln mit einem stark sauren Ionenaustauscher in der $H^+$-Form (Dowex 50 W X8) und einem stark basischen Ionenaustauscher in der $HO^-$-Form (Dowex I), jeweils in einer Säule von 180 mm × 30 mm, entsalzt. Die nunmehr neutrale Lösung wurde unter vermindertem Druck zur Trockne eingedampft. Der Rückstand wurde mehrmals in absolutem Ethanol aufgenommen und wieder eingedampft, um restliches Wasser zu entfernen, und schließlich mit absolutem Diethylether angerieben. Die zunächst erhaltenen 3,67 g (R)-4-Hydroxy-2-pyrrolidon wurden im Hochvakuum in einem Kugelrohr bei einer Luftbadtemperatur von 150 bis 160°C sublimiert. Es ergaben sich 3,60 g (89% der Theorie, bezogen auf eingesetztes (2S, 4R)-4-Hydroxyprolinmethylester-hydrochlorid) farblose Kristalle mit einem Schmelzpunkt von 156 - 158°C und einem $[\alpha]_D^{20}$ von +59,5° (c = 1,14, $H_2O$).

Reaktionsstufe c):

Eine Lösung von 3,60 g (35,6 mMol) (R)-4-Hydroxy-2-pyrrolidon in 60 ml 4N Salzsäure wurde 4 Stunden lang unter Rückfluß erhitzt und dann unter vermindertem Druck fast vollständig eingedampft. Danach wurde der Rückstand durch mehrmaliges Lösen in Wasser und erneutes Eindampfen von überschüssiger Salzsäure befreit. Dann wurde der Rückstand wieder in Wasser gelöst und über eine mit einem stark sauren Ionenaustauscher in der $H^+$-Form (Dowex 50 W X8) gefüllte Säule von 180 mm × 30 mm gegeben. Der Ionenaustauscher wurde mit einer 1N wäßrigen Ammoniak-Lösung eluiert, das Eluat wurde unter vermindertem Druck zur Trockne eingedampft und der Rückstand wurde unter Zusatz von Aktivkohle aus einem Wasser/Ethanol-Gemisch umkristallisiert. Es wurden 3,98 g (94% der Theorie) an farbloser (R)-4-Amino-3-hydroxybutansäure mit einem Schmelzpunkt von 213 - 215°C (Zersetzung) und einem $[\alpha]_D^{20}$ von -20,5° (c = 2,08, $H_2O$) erhalten.

Die Gesamtausbeute, bezogen auf das ursprünglich eingesetzte (2S, 4R)-4-Hydroxyprolinmethylester-hydrochlorid, betrug somit ca. 84% der Theorie.

## Ansprüche

1. Verfahren zur Herstellung von (R)-4-Amino-3-hydroxybutansäure, dadurch gekennzeichnet, daß man a) ein (2S, 4R)-4-Hydroxyprolinester-hydrochlorid der Formel

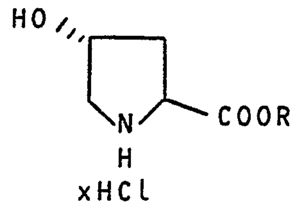

(I),

in der R einen Methyl-oder Ethylrest bedeutet, durch Behandeln in wasserfreiem Medium mit zuerst einem Alkalimetallmethoxid oder -ethoxid, dann t-Butylhypochlorit und schließlich Triethylamin in den (R)-4-Hydroxy-1-pyrrolin-2-carbonsäureester der Formel

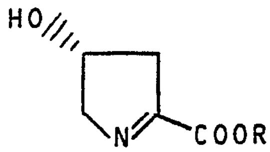

(II),

in der R wieder die bereits angegebene Bedeutung hat, umwandelt,

b) diesen ohne weitere Reinigung in einer Alkalimetallhydroxid-Lösung auflöst, die Lösung mit Wasserstoffperoxid versetzt, das Reaktionsgemisch durch langsame Zugabe einer Mineralsäure ansäuert und anschließend entsalzt und durch Einengen das gebildete (R)-4-Hydroxy-2-pyrrolidon der Formel

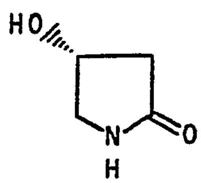

(III)

gewinnt und

c) dieses mit einer Mineralsäure zu (R)-4-Amino-3-hydroxybutansäure der Formel

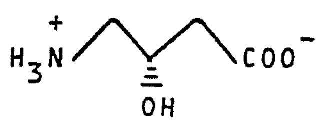

(IV)

hydrolysiert.

2. Verfahren nach Anspruch 1, dadurch gekennzeichnet, daß man in der Reaktionsstufe a) die Behandlung mit dem Alkalimetallmethoxid oder -ethoxid in dem entsprechenden Alkohol,die Behandlung mit dem t-Butylhypochlorit und dem Triethylamin in absolutem Tetrahydrofuran oder 1,2-Dimethoxyethan oder in einer Mischung aus einem dieser Ether und absolutem Diethylether vornimmt.

3. Verfahren nach Anspruch 1 oder 2, dadurch gekennzeichnet, daß man in der Reaktionsstufe a) das Alkalimetallmethoxid oder -ethoxid, das t-Butylhypochlorit und das Triethylamin jeweils in der 1-bis 1,05-fachen molaren Menge des eingesetzten (2S, 4R)-4-Hydroxyprolinester-hydrochlorids einsetzt.

4. Verfahren nach einem der Ansprüche 1 bis 3, dadurch gekennzeichnet, daß man in der Reaktionsstufe a) die Behandlung mit dem Alkalimetallmethoxid oder -ethoxid bei einer Temperatur zwischen 10 und 30°C, die Behandlung mit dem t-Butylhypochlorit bei einer Temperatur zwischen -40 und -20°C und die Behandlung mit dem Triethylamin bei einer Temperatur zwischen -20 und +10°C vornimmt.

5. Verfahren nach einem der Ansprüche 1 bis 4, dadurch gekennzeichnet, daß man in der Reaktionsstufe b) das Alkalimetallhydroxid und das Wasserstoffperoxid jeweils in der 1-bis 1,05-fachen molaren Menge des ursprünglich eingesetzten (2S, 4R)-4-Hydroxyprolinester-hydrochlorids und die Mineralsäure in einer dem Alkalimetallhydroxid mindestens äquivalenten Menge einsetzt.

6. Verfahren nach einem der Ansprüche 1 bis 5, dadurch gekennzeichnet, daß man die Reaktionsstufe b) bei einer Temperatur zwischen 20 und 50 °C vornimmt.

7. Verfahren nach einem der Ansprüche 1 bis 6, dadurch gekennzeichnet, daß man in der Reaktionsstufe c) die Mineralsäure in der 5-bis 10-fachen molaren Menge des eingesetzten (R)-4-Hydroxy-2-pyrrolidons einsetzt.

8. Verfahren nach einem der Ansprüche 1 bis 7, dadurch gekennzeichnet, daß man die Reaktionsstufe c) bei einer Temperatur zwischen 90°C und der Rückflußtemperatur des Reaktionsgemisches vornimmt.